# EUROPEAN PATENT APPLICATION

(11) **EP 2 017 608 A1**
(43) Date of publication of application: **21.01.2009**
(21) Application number: 07741963.8
(22) Date of filing: 19.04.2007
(51) Int. Cl.: G01N 27/327, G01N 27/416

(54) **BIOSENSOR**

(30) Priority: 19.04.2006 JP 2006116176
(71) Applicant: Panasonic Corporation, Kadoma-shi Osaka 571-8501 (JP)
(72) Inventor: NAKAYAMA, Junko c/o Panasonic Corp., IPROC, IP Development Center, Shiromi, Chuo-ku, Osaka-shi, Osaka 540-6207 (JP); TAKAHARA, Yoshifumi c/o Panasonic Corp., IPROC, IP Development Center, Shiromi, Chuo-ku, Osaka-shi, Osaka 540-6207 (JP); YAMANISHI, Eriko Junko c/o Panasonic Corp., IPROC, IP Development Center, Shiromi, Chuo-ku, Osaka-shi, Osaka 540-6207 (JP); ITOH, Yoshihiro c/o Panasonic Corp., IPROC, IP Development Center, Shiromi, Chuo-ku, Osaka-shi, Osaka 540-6207 (JP)
(74) Representative: Pautex Schneider, Nicole Véronique
(86) International application number: PCT/JP2007/058527
(87) International publication number: WO 2007/123179

(57) **Abstract**

In a biosensor for measuring glucose in a liquid sample, an additive agent which is one of an organic acid or organic acid salt having at least one carboxyl group in a molecule, an organic acid or organic acid salt having at least one amino group or carbonyl group in a molecule, a sugar alcohol, and a solubilized protein, or a combination thereof is added into a reagent layer including glucose dehydrogenase having flavin adenine dinucleotide as a coenzyme, which is provided on an insulating substrate. Thereby, the substrate specificity to glucose and the preservation stability can be enhanced, and further, reactions to sugars other than glucose can be avoided.

## Description

### TECHNICAL FIELD

The present invention relates to a biosensor for analyzing a specific component in a sample solution, and more particularly, to a reagent formulation for composing a reagent layer of the biosensor.

### BACKGROUND ART

A biosensor is a sensor which utilizes the molecule identifying abilities of biological materials such as microorganisms, enzymes, and antibodies to apply the biological materials as molecule recognition elements. To be specific, the biosensor utilizes a reaction which occurs when an immobilized biological material recognizes a target specific component, such as oxygen consumption by respiration of a micro-organism, an enzyme reaction, or luminescence.

Among biosensors, enzyme sensors have been advanced in practical applications, and particularly, enzyme sensors for glucose are utilized in observing the medical conditions of diabetes. As an example of such enzyme sensor for glucose, a biosensor proposed in Patent Document 1 has been disclosed.

This biosensor is obtained by forming an electrode layer comprising a measurement electrode, a counter electrode, and a detection electrode on an insulating substrate, and forming a reagent layer including an enzyme or the like which reacts specifically with a specific component in a sample solution on the electrode layer. As an enzyme included in the reagent layer, glucose dehydrogenase having pyrrolo-quinoline quinone as a coenzyme (hereinafter referred to as PQQ-GDH) is adopted. Further, the reagent layer includes an electron acceptor such as potassium ferricyanide, besides the enzyme PQQ-GDH.

When blood is applied to the biosensor of this configuration, the enzyme PQQ-GDH reacts with glucose in blood on the reagent layer to generate gluconolactone and electrons, and the ferricyanide ion as the electron acceptor is reduced to the ferrocyanide ion by the generated electrons. A constant voltage is applied thereto to again oxidize the ferrocyanide ion to the ferricyanide ion. The glucose concentration (blood sugar level) in blood can be measured from the value of current that occurs at this time.
Patent Document 1: Japanese Published Patent Application No. 2000-171428
Patent Document 2: Japanese Published Patent Application No. 2001-343350
Patent Document 3: Japanese Published Patent Application No. 2002-207022

### DISCLOSURE OF THE INVENTION

### PROBLEMS TO BE SOLVED BY THE INVENTION

However, the enzyme PQQ-GDH used in the conventional biosensor reacts not only with glucose but also with a sugar other than substrate, such as maltose. Therefore, if a patient who is being administered an infusion including such as icodextrin or maltose measures his blood sugar level using this biosensor, a value higher than the actual blood sugar level might be indicated. If administration of excessive insulin is performed based on such measured value, a medical accident might occur.

The present invention is made to solve the above-described problems and has for its object to provide a biosensor capable of performing highly precise measurement, which is excellent in the substrate specificity to glucose, and can avoid actions to sugars other than glucose.

### MEASURES TO SOLVE THE PROBLEMS

In order to solve the above-described problems, according to Claim 1 of the present invention, there is provided a biosensor which, having a reagent layer including a reagent which reacts specifically with a specific component in a sample solution, measures the concentration of the specific component in the sample solution, and the reagent layer includes glucose dehydrogenase having flavin adenine dinucleotide as a coenzyme, and an organic acid or its salt having at least one carboxyl group in a molecule thereof.

According to Claim 2 of the present invention, in the biosensor defined in Claim 1, the concentration of the specific component in the sample solution is measured using electrodes including at least a working electrode and a counter electrode, which electrodes are formed on an insulating substrate.

According to Claim 3 of the present invention, in the biosensor defined in Claim 2, the reagent layer, including an electron carrier, is formed on the electrodes.

According to Claim 4 of the present invention, in the biosensor defined in Claim 2, the reagent layer, including an electron carrier, is formed so that the electrodes are disposed within a diffusion area wherein the reagent of the reagent layer is dissolved in the sample solution and diffused.

According to Claim 5 of the present invention, in the biosensor defined in Claim 1, the organic acid is aliphatic carboxylic acid, carbocyclic carboxylic acid, or heterocyclic carboxylic acid, or a substitution or derivative thereof.

According to Claim 6 of the present invention, in the biosensor defined in Claim 5, the organic acid or its salt is any of citric acid, citrate, phthalic acid, and phthalate, or a combination of these.

According to Claim 7 of the present invention, there is provided a biosensor which, having a reagent layer including a reagent which reacts specifically with a specific component in a sample solution, measures the concentration of the specific component in the sample solution, and the reagent layer includes glucose dehydrogenase having flavin adenine dinucleotide as a coenzyme, and an organic acid or its salt having at least one amino group or carbonyl group in a molecule thereof.

According to Claim 8 of the present invention, in the biosensor defined in Claim 7, the concentration of the specific component in the sample solution is measured using electrodes including at least a working electrode and a counter electrode, which electrodes are formed on an insulating substrate.

According to Claim 9 of the present invention, in the biosensor defined in Claim 8, the reagent layer, including an electron carrier, is formed on the electrodes.

According to Claim 10 of the present invention, in the biosensor defined in Claim 8, the reagent layer, including an electron carrier, is formed so that the electrodes are disposed in a diffusion area wherein the reagent of the reagent layer is dissolved in the sample solution and diffused.

According to Claim 11 of the present invention, in the biosensor defined in Claim 7, the organic acid is amino acid, or a substitution or derivative thereof.

According to Claim 12 of the present invention, in the biosensor defined in Claim 11, the organic acid is taurine.

According to Claim 13 of the present invention, there is provided a biosensor which, having a reagent layer including a reagent which reacts specifically with a specific component in a sample solution, measures the concentration of the specific component in the sample solution, and the reagent layer includes glucose dehydrogenase having flavin adenine dinucleotide as a coenzyme, and a sugar alcohol.

According to Claim 14 of the present invention, in the biosensor defined in Claim 13, the concentration of the specific component in the sample solution is measured using electrodes including at least a working electrode and a counter electrode, which electrodes are formed on an insulating substrate.

According to Claim 15 of the present invention, in the biosensor defined in Claim 14, the reagent layer, including an electron carrier, is formed on the electrodes.

According to Claim 16 of the present invention, in the biosensor defined in Claim 14, the reagent layer, including an electron carrier, is formed so that the electrodes are disposed in a diffusion area wherein the reagent of the reagent layer is dissolved in the sample solution and diffused.

According to Claim 17 of the present invention, in the biosensor defined in Claim 13, the sugar alcohol is chain polyhydric alcohol or cyclic sugar alcohol, or a substitution or derivative thereof.

According to Claim 18 of the present invention, in the biosensor defined in Claim 17, the sugar alcohol is either or both of maltitol and lactitol.

According to Claim 19 of the present invention, there is provided a biosensor which, having a reagent layer including a reagent which reacts specifically with a specific component in a sample solution, measures the concentration of the specific component in the sample solution, and the reagent layer includes glucose dehydrogenase having flavin adenine dinucleotide as a coenzyme, and a solubilized protein.

According to Claim 20 of the present invention, in the biosensor defined in Claim 19, the concentration of the specific component in the sample solution is measured using electrodes including at least a working electrode and a counter electrode, which electrodes are formed on an insulating substrate.

According to Claim 21 of the present invention, in the biosensor defined in Claim 20, the reagent layer, including an electron carrier, is formed on the electrodes.

According to Claim 22 of the present invention, in the biosensor defined in Claim 20, the reagent layer, including an electron carrier, is formed so that the electrodes are disposed in a diffusion area wherein the reagent of the reagent layer is dissolved in the sample solution and diffused.

According to Claim 23 of the present invention, in the biosensor defined in Claim 19, the solubilized protein is bovine serum albumin (BSA), egg albumin, gelatin, or collagen.

According to Claim 24 of the present invention, there is provided a biosensor which, having a reagent layer including a reagent which reacts specifically with a specific component in a sample solution, measures the concentration of the specific component in the sample solution, and the reagent layer includes glucose dehydrogenase having flavin adenine dinucleotide as a coenzyme, and at least two additive agents among an organic acid or its salt having at least one carboxyl group in a molecule thereof, an organic acid or its salt having at least one amino group or carbonyl group in a molecule thereof, a sugar alcohol, and a solubilized protein.

According to Claim 25 of the present invention, in the biosensor defined in Claim 24, the concentration of the specific component in the sample solution is measured using electrodes including at least a working electrode and a counter electrode, which electrodes are formed on an insulating substrate.

According to Claim 26 of the present invention, in the biosensor defined in Claim 25, the reagent layer, including an electron carrier, is formed on the electrodes.

According to Claim 27 of the present invention, in the biosensor defined in Claim 25, the reagent layer, including an electron carrier, is formed so that the electrodes are disposed in a diffusion area wherein the reagent of the reagent layer is dissolved in the sample solution and diffused.

### EFFECTS OF THE INVENTION

According to the biosensor of the present invention, since an organic acid or an organic acid salt including at least a carboxyl group is added to the reagent layer including FAD-GDH, the blanking current value can be suppressed without blocking an enzyme reaction or the like, and further, unnecessary reactions with various foreign substances existing in blood can also be suppressed, thereby realizing a biosensor which has a favorable linearity, less variations among individual sensors, and an excellent substrate specificity to glucose, and can perform highly precise measurement.

Further, according to the biosensor of the present invention, since an organic acid or an organic acid salt having at least an amino group or a carbonyl group is added to the reagent layer including FAD-GDH, the reagent layer can be densely and homogeneously formed, resulting in a biosensor which is excellent in substrate specificity to glucose and can perform highly precise measurement, and which thereby can dramatically improve the responsivity of the sensor to the glucose concentration.

Further, according to the biosensor of the present invention, since a sugar alcohol is added into the reagent layer including FAD-GDH, the blanking current value can be suppressed without blocking the enzyme reaction or the like, thereby realizing a biosensor which has a favorable linearity, less variations among individual sensors, excellent substrate specificity to glucose, and excellent preservation stability, and can perform highly precise measurement.

Further, according to the biosensor of the present invention, since a solubilized protein is added to the reagent layer including FAD-GDH, it is possible to realize a biosensor which is excellent in preservation stability and substrate specificity to glucose and can perform highly precise measurement, and which thereby can reduce the influences by hematocrit and ambient temperature without blocking the enzyme reaction or the like as well as can reduce unnecessary reactions with various foreign substances existing in blood.

### BRIEF DESCRIPTION OF THE DRAWINGS

Figure 1 is a diagram illustrating a constitutional example of a biosensor of the present invention.
Figure 2 is a diagram illustrating another constitutional example of a biosensor of the present invention.
Figure 3 is a diagram illustrating the sensor response characteristics in the case where maltose is added as a sample solution.
Figure 4 is a diagram illustrating the sensor response characteristics in the case where glucose is added as a sample solution.
Figure 5(a) is a diagram illustrating a blanking value in the case where purified water is used as a sample solution, figure 5(b) is a diagram illustrating a blanking value in the case where an organic acid is used as a reagent and purified water is added as a sample solution, and figure 5(c) is a diagram showing the sensor response characteristics in the case where glucose is added as a sample solution.
Figure 6 is a diagram illustrating the response characteristics of the current value due to an addition of taurine as an example of an amino acid in the sensor using whole blood as a sample solution.
Figure 7(a) is a diagram illustrating the preservation characteristics under a hot and humid environment in the case where whole blood is used as a sample solution and a sugar alcohol is added to the reagent layer, figure 7(b) is a diagram illustrating the response characteristics of background current, and figure 7(c) is a diagram illustrating the response characteristics of current.
Figure 8(a) is a diagram illustrating the influence by hematocrit to the sensor response characteristics in the case where whole blood is used as a sample solution, and figure 8(b) is a diagram illustrating the influence by hematocrit to the sensor response characteristics in the case where BSA is added into the reagent layer.

### DESCRIPTION OF REFERENCE NUMERALS

- 1: substrate
- 2: working electrode
- 3: counter electrode
- 4: detection electrode
- 5: reagent layer
- 6: spacer
- 6a: notch
- 7: cavity
- 8: cover
- 9: air hole
- 10,11,12: leads
- 101: substrate
- 102: working electrode
- 103: counter electrode
- 105: reagent layer
- 106: spacer
- 106a: notch
- 107: cavity
- 108: cover
- 109: air hole
- 110,111: leads

### BEST MODE TO CARRY OUT THE INVENTION

Hereinafter, preferred embodiments of the present invention will be described with reference to the drawings.

### (Embodiment 1)

Hereinafter, a biosensor according to a first embodiment of the present invention will be described.

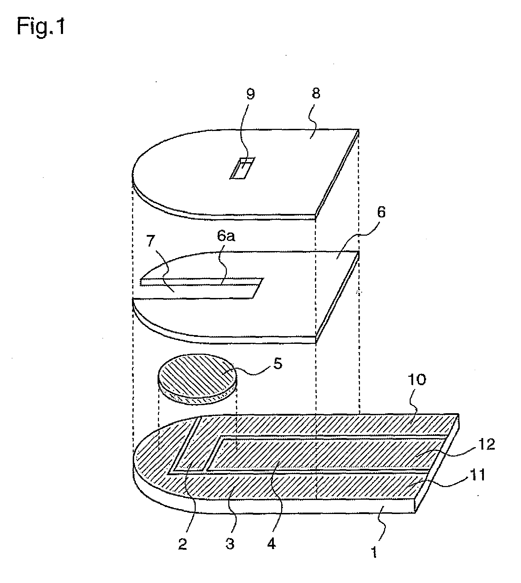

Figure 1 is a diagram illustrating an example of a configuration of a three-electrode-system biosensor according to the first embodiment.

The three-electrode-system biosensor shown in figure 1 is provided with an insulating substrate 1 having an electric conductive layer on its surface, a reagent layer 5, a spacer 6 having a notch 6a, and a cover 8 having an air hole 9.

As a material of the insulating substrate 1, there may be adopted polyethylene terephthalate, polycarbonate, polyimide, or the like.

As a material of the electric conductive layer, there may be adopted a single substance such as a carbon or a noble metal like gold, platinum, or palladium, or a complex substance such as a carbon paste or a noble metal paste.

As a material of the spacer 6 and the cover 8, there may be adopted polyethylene terephthalate, polycarbonate, polyimide, polybutylene terephthalate, polyamide, polyvinyl chloride, polyvinylidene chloride, nylon, or the like.

A description will be given of a fabrication method for the biosensor of such configuration.

After an electric conductive layer is formed on the insulating substrate 1 by a sputtering deposition method or a screen printing method, slits are formed using laser or the like, thereby producing a working electrode 2, a counter electrode 3, and a detection electrode 4. The detection electrode 4 functions not only as an electrode for detecting a shortage of the analyte amount, but it can be used as a part of the reference electrode or the counter electrode.

Then, a reagent layer 5 including glucose dehydrogenase having flavin adenine dinucleotide as a coenzyme (hereinafter referred to as FAD-GDH), an electron carrier, and an additive agent is formed on the electrodes 2, 3 and 4. Thereafter, a spacer 6 and a cover 8 are bonded together onto the reagent layer 5 and the electrodes 2, 3, and 4, thereby forming a cavity 7 into which a sample solution is supplied. While the supply of the sample solution into the biosensor is realized by a capillary phenomenon, the capillary phenomenon is promoted by providing the cover 8 with an air hole 9 for letting the air in the cavity 7 out of the biosensor, whereby the supply of the sample solution can be smoothly carried out.

The sample solution is supplied from the inlet of the cavity 7 into the cavity 7 by the capillary phenomenon, and when it reaches the position of the reagent layer 5, a specific component in the sample solution reacts with the reagent included in the reagent layer 5. The amount of change in current which occurs due to this reaction is read with an external measurement device which is connected through the leads 10, 11, and 12 of the working electrode 2, the counter electrode 3, and the detection electrode 4, respectively, thereby determining the quantity of the specific component in the sample solution.

Hereinafter, the reagent components included in the reagent layer 5 will be described.

Initially, the enzyme will be described.

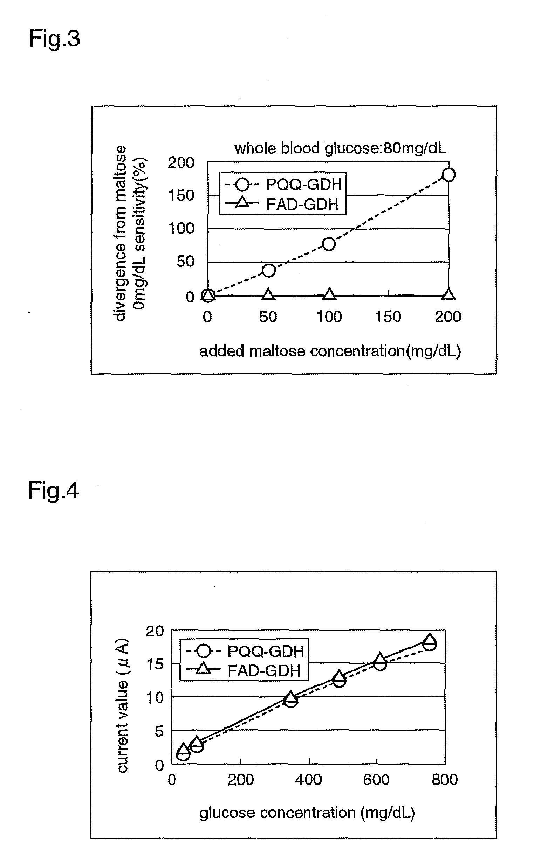

In this first embodiment, FAD-GDH is adopted as an enzyme to be included in the reagent layer 5. Hereinafter, the reason why FAD-GDH is adopted will be described with reference to figures 3 and 4.

Figure 3 shows the sensor response characteristics in the case where maltose in a concentration range of 0 to 200mg/dL is added under the presence of a glucose concentration of 80mg/dL. In figure 3, the ordinate shows the reactivities of PQQ-GDH and FAD-GDH to maltose (the divergence rates (%) from maltose 0mg/dL sensitivity), and the abscissa shows the added maltose concentration (mg/dL).

PQQ-GDH is an enzyme used in the conventional biosensor, and its sensor response value increases in proportion to the additive amount of maltose. On the other hand, FAD-GDH is an enzyme adopted in the biosensor of this first embodiment, and its sensor response value is as low as the value when no maltose is added even when the additive amount of maltose is increased, and thus it is found that the reactivity to maltose is low.

Figure 4 shows the sensor response characteristics in the case where glucose is added as a sample solution. In figure 4, the ordinate shows the sensor response value (current value (µ A)), and the abscissa shows the glucose concentration (mg/dL).

When PQQ-GDH is used as an enzyme, the sensor response value increases in proportion to the glucose concentration. When FAD-GDH is used as an enzyme, the sensor response value increases in proportion to the glucose concentration like in the case of using PQQ-GDH. Accordingly, the reactivities of the both enzymes to glucose are approximately the same.

From the aforementioned result, it is found that the enzyme FAD-GDH adopted in this first embodiment has a low reactivity to maltose while having approximately the same substrate specificity to glucose as that of the enzyme PQQ-GDH adopted in the conventional biosensor.

Accordingly, in this first embodiment, since FAD-GDH is adopted as an enzyme, it is possible to realize an excellent biosensor which is excellent in the substrate specificity to glucose and has a low reactivity to maltose, that is, which is favorably correlated with glucose and is not affected by the influence by maltose.

Next, the additive agent will be described.

In this first embodiment, an organic acid or an organic acid salt having at least one carboxyl group in a molecule is adopted as an additive agent to be added to the reagent layer 5.

The organic acid or organic acid salt having at least one carboxyl group in a molecule functions to prevent that the oxidized-form electron carrier contacts with some highly-reactive functional groups which exist in the enzyme protein included in the reagent and thereby the electron carrier is denatured (reduced) from the oxidized form to the reduced form.

Therefore, a noise current which occurs due to the reduction reaction between the FAD-GDH and the electron carrier included in the reagent layer 5 under the existence of heat and moisture can be suppressed by adding the organic acid or organic acid salt having at least one carboxyl group in a molecule into the reagent layer 5, thereby preventing deterioration of the performance of the biosensor, and increasing the preservation stability. Further, since unnecessary reactions with various foreign substances existing in blood, particularly in blood cells, can be also suppressed, variations among individual sensors can be suppressed, and a favorable linearity can be realized, that is, the slope of the regression formula is increased while the intercept thereof is decreased, and thus highly precise measurement can be carried out.

The organic acid or organic acid salt having at least one carboxyl group in a molecule may be aliphatic carboxylic acid, carbocyclic carboxylic acid, heterocyclic carboxylic acid, or their salts.

For example, the aliphatic carboxylic acid may be malonic acid, succinic acid, glutaric acid, adipic acid, maleic acid, fumaric acid, or their salts. The degree of effect becomes larger as the straight chain is longer and the molecular weight is larger, and particularly, one having three or more hydrocarbon chins is desirable. Further, since the reagent used in the biosensor is required to have a high solubility in water, one having more hydrophilic functional groups in the molecular structure is more desirable.

The carbocyclic carboxylic acid may be acidum benzoicum, phthalic acid, isophthalic acid, terephthalic acid, or their salts, and the same effects as described above can be achieved by using these materials.

The heterocyclic carboxylic acid may be 2-furoic acid, nicotinic acid, isonicotinic acid, or their salts, and the same effects as described above can be achieved by using these materials.

Besides the above-described aliphatic or carbocyclic carboxylic acid, and carboxylic acid or carboxylate salt having a heterocyclic ring, there may be adopted, for example, malic acid, oxaloacetic acid, citric acid, ketoglutaric acid and their salts in which functional groups of the carboxylic acid or carboxylate salt are partially replaced with other functional groups, with the same effect as described above.

Among these organic acids or organic acid salts, glutaric acid, adipic acid, phthalic acid, and acidum benzoicum are most suitable.

The additive amounts of these organic acids or organic acid salts are preferably in a range of 0.0005 to 100mM as the reagent solution concentration to the enzyme solution concentration of 500 to 2500U/ml.

According to the biosensor of this first embodiment, since the FAD-GDH having an excellent substrate specificity to glucose and the organic acid or organic acid salt having at least a carboxyl group are included in the reagent layer 5, the blanking current value can be suppressed without blocking the enzyme reaction and the like, and further, unnecessary reactions with various foreign substances existing in blood can also be suppressed, whereby the substrate specificity to glucose as well as the preservation stability can be enhanced, and thus highly precise measurement can be performed.

### (Embodiment 2)

Hereinafter, a biosensor according to a second embodiment of the present invention will be described.

The biosensor of this second embodiment is characterized by that the reagent layer 5 shown in figure 1 includes FAD-GDH, an electron carrier, and an organic acid or an organic acid salt having at least one amino group or carbonyl group in a molecule. Since other constituents are identical to those of the biosensor of the first embodiment, repeated description is not necessary.

Hereinafter, an additive agent will be described.

In this second embodiment, an organic acid or an organic acid salt having at least one amino group or carbonyl group in a molecule is used as an additive agent to be added to the reagent layer 5.

The organic acid or organic acid salt having at least one amino group or carbonyl group in a molecule can make the surface state of the reagent layer 5 very smooth and homogeneous.

Although the reagent layer is likely to be crystallized in the process of during the reagent solution particularly when an inorganic salt such as potassium ferricyanide used as an electron carrier is included in the reagent layer, such crystalline growth of the inorganic salt can be prevented by including the organic acid or organic acid salt having at least one amino group or carbonyl group in a molecule, in the reagent layer 5.

Since the inorganic salt whose crystalline growth is prevented exists in its particulate state in the reagent layer 5, it can closely and uniformly contact with the enzyme molecule, thereby realizing a reagent layer condition having a favorable electron transfer efficiency with the enzyme molecule. Further, since the solubility of the reagent layer can be enhanced, the sensitivity and linearity of the sensor can be dramatically enhanced.

As the organic acid or organic acid salt having at least one amino group or carbonyl group in a molecule, there may be adopted amino acids such as glycine, alanine, valine, leucine, isoleucine, serine, threonine, methionine, asparagine, glutamine, arginine, lycine, histidine, phenylalanine, triptophane, and proline, or their salts, or sarcosine, betaine, and taurine, or their substitions, derivatives, and salts. Among these amino acids, substitutions, derivatives, and their salts, particularly glycine, serine, proline, threonine, lycine, and taurine are preferable because of their high crystallization blocking effects.

The additive amounts of these amino acid or their substitutions, derivatives, and salts are preferably 10 to 100mM as the reagent solution concentration to the enzyme solution concentration of 500 to 2500U/ml.

According to the biosensor of this second embodiment, since the FAD-GDH having an excellent substrate specificity to glucose and an organic acid or an organic acid salt having at least an amino group or a carbonyl group are included in the reagent layer 5, the reagent layer can be formed densely and homogeneously, and the responsivity of the sensor to the glucose concentration can be dramatically enhanced, thereby realizing highly precise measurement.

### (Embodiment 3)

Hereinafter, a biosensor according to a third embodiment of the present invention will be described.

The biosensor of this third embodiment is characterized by that the reagent layer 5 shown in figure 1 includes FAD-GDH, an electron carrier, and a sugar alcohol. Since other constituents are identical to those of the biosensor of the first embodiment, repeated description is not necessary.

Hereinafter, an additive agent will be described.

In this third embodiment, a sugar alcohol is adopted as an additive agent to be added to the reagent layer 5.

The sugar alcohol functions to prevent that the oxidized-form electron carrier contacts with some highly-reactive functional groups existing in the enzyme protein included in the reagent and thereby the electron carrier is denatured (reduced) from the oxidized-form to the reduced-form.

Therefore, a noise current which occurs due to the reduction reaction between the FAD-GDH and the electron carrier included in the reagent layer 5 under the existence of heat and moisture can be suppressed by adding the sugar alcohol into the reagent layer 5, and thereby the performance of the biosensor is prevented from being deteriorated. Further, since unnecessary reactions with various foreign substances existing in blood, particularly in blood cells, can be also suppressed, a favorable linearity is obtained, and variations among individual sensors can be suppressed.

As the sugar alcohol, there may be adopted chain polyhydric alcohols or cyclic sugar alcohols such as sorbitol, maltitol, xylitol, mannitol, lactitol, palatinit, arabinitol, glycerol, ribitol, galactitol, sedoheptitol, perseitol, boremitol, styratitol, polygalitol, iditol, talitol, allitol, ishylitol, reduced starch saccharified material, and ishylitol. The same effects can also be achieved by using the stereoisomers, substitutions, or derivatives of these sugar alcohols.

Among these sugar alcohols, maltitol and lactitol are the most suitable materials because they are relatively low in the unit price, are easily available, and are highly effective in suppressing the noise current.

The additive amounts of these sugar alcohols are preferably 0.1 to 50mB as the reagent solution concentration to the enzyme solution concentration of 500 to 2500U/ml.

According to the biosensor of this third embodiment, since the FAD-GDH having an excellent substrate specificity to glucose and the sugar alcohol are included in the reagent layer 5, the blanking current value can be suppressed without blocking the enzyme reaction or the like, and further, unnecessary reactions with various foreign substances existing in blood can also be suppressed, whereby the substrate specificity to glucose and the preservation stability can be enhanced, and thus highly precise measurement can be performed.

### (Embodiment 4)

Hereinafter, a biosensor according to a fourth embodiment of the present invention will be described.

The biosensor of this fourth embodiment is characterized by that the reagent layer 5 shown in figure 1 includes FAD-GDH, an electron carrier, and a solubilized protein. Since other constituents are identical to those of the biosensor of the first embodiment, repeated description is not necessary.

Hereinafter, an additive agent will be described.

In this fourth embodiment, a solubilized protein is adopted as an additive agent to be added to the reagent layer 5.

The solubilized protein has a variety of functions such as transfer of agents and the like, maintenance of osmotic pressure, and maintenance of electrolyte balance, without blocking the enzyme reaction or the like.

Therefore, by adding the solubilized protein into the reagent layer 5, supply to the vicinity of the glucose electrode can be restricted without blocking the enzyme reaction or the like, thereby reducing the influence by hematocrit and the influence by ambient temperature.

The solubilized protein may be bovine serum albumin (BSA), egg albumin, gelatin, collagen or the like.

The additive amounts of these solubilized protein are preferably 0.01 to 1.00wt% to the enzyme solution concentration of 500 to 2500U/ml.

According to the biosensor of this fourth embodiment, since the FAD-GDH having an excellent substrate specificity to glucose and the solubilized protein are included in the reagent layer 5, the influence by hematocrit and the influence by ambient temperature can be reduced without blocking the enzyme reaction or the like, and thereby the preservation stability and the substrate specificity to glucose can be enhanced, resulting in highly precise measurement.

While in the first to fourth embodiments the description is given of the case where an organic acid or organic acid salt having at least one carboxyl group in a molecule, an organic acid or organic acid salt having at least one amino group or carboxyl group in a molecule, a sugar alcohol, or a solubilized protein is respectively added to the reagent layer 5 as an additive agent, these additive agents may be appropriately combined.

Further, in the first to fourth embodiments, as the electron carrier included in the reagent, there may be adopted potassium ferricyanide, p-benzoquinone and its derivative, or phenazine methosulfate, methylene blue, ferrocene and its derivative.

Furthermore, while in the first to fourth embodiments the description is given of the case where the reagent layer 5 is provided on the electrodes, specifically the reagent layer 5 may be arranged over the entire surface or part of the electrodes. Alternatively, the reagent layer 5 may be arranged such that the electrodes are disposed within a range wherein the performance of the biosensor is not deteriorated, i.e., within a diffusion area wherein the reagent in the reagent layer is dissolved in the sample solution and diffused.

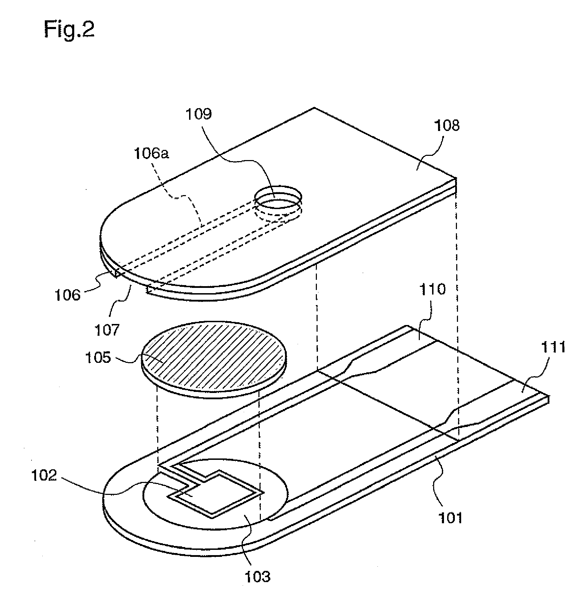

Further, while in the first to fourth embodiments the three-electrode-system biosensors are described, a two-electrode-system biosensor may be fabricated. The configuration of the two-electrode-system biosensor is shown in figure 2.

The two-electrode-system biosensor shown in figure 2 includes an insulating substrate 101, a reagent layer 105, a spacer 106, and a cover 108. The materials of the respective constituents may be identical to those of the constituents shown in figure 1.

A method of fabricating such biosensor will be described.

After an electric conductive layer is formed on an insulating substrate 101 by a sputtering deposition method or a screen printing method, slits are formed using laser or the like to form a working electrode 102 and a counter electrode 103. A reagent layer 105 including FAD-GDH, an electron carrier, and an additive agent is formed on the electrodes. Then, a spacer 106 having a notch 106a and a cover 108 are bonded together on the reagent layer 105 and the electrodes 102 and 103, thereby producing a cavity 107 into which a sample solution is supplied.

The sample solution is supplied from an inlet of the cavity 107 into the cavity 107 by a capillary phenomenon, and when it reaches the position of the reagent layer 5, a specific component in the sample solution reacts with the reagent included in the reagent layer 105. The amount of change in the current that occurs due to this reaction is read by an external measurement device which is connected through the leads 110 and 111 of the working electrode 102 and the counter electrode 103, thereby determining the quantity of the specific component in the sample solution.

### (Example 1)

An electrode layer comprising a working electrode 102 and a counter electrode 103 is formed on an insulating substrate 101 comprising polyethylene terephthalate by screen printing. A reagent layer 105 including an enzyme (FAD-GDH: 600 to 1500U/ml), an electron carrier (potassium ferricyanide), an amino acid (taurine: 50 to 85mM), and a sugar alcohol (maltitol: 1 to 3mM) is formed on the electrode layer. A spacer 106 comprising polyethylene terephthalate and a cover 108 comprising polyethylene terephthalate are formed on the reagent layer 105. Thus, a two-electrode-system blood sugar level measuring sensor is fabricated.

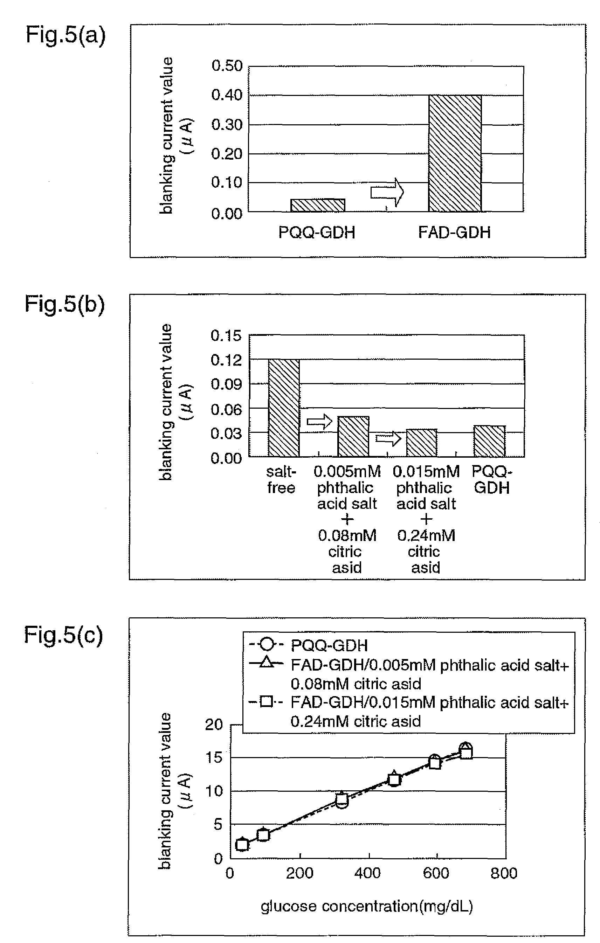

A sensor response value (blanking current value) in the case where purified water is used as a sample solution in the fabricated blood sugar level measurement sensor is measured. The result is shown in figure 5(a). For comparison, figure 5(a) also shows the sensor response characteristics of the conventional biosensor in which PQQ-GDH is adopted as an enzyme, and citrate (0.05 to 0.15mM), taurine (50 to 85mM), and maltitol (1 to 3 mM) are added into the regent layer to the enzyme solution concentration of 1000 to 1500U/ml.

As is evident from figure 5(a), the blanking current value of the biosensor using FAD-GDH of the present invention is as high as 0.40µA while the blanking current value of the conventional biosensor using PQQ-GDH is about 0.03µ A. Since accurate quantitative determination of glucose cannot be performed when the blanking current value is high, the blanking current value must be reduced. So, an organic acid having a function of suppressing a noise current is added to the reagent layer. To be specific, phthalate salt and citric salt are added to the reagent layer with their concentrations being adjusted to 0.05mM and 0.08mM with respect to the FAD-GDH enzyme solution concentration of 1500U/ml, respectively, whereby the blanking current value is reduced as compared with when it is not added as shown in figure 5(b). Further, when phthalate salt and citric salt are added with their concentrations being adjusted to 0.015mM and 0.24mM, respectively, the blanking current value is more reduced.

The sensor response current value to the glucose concentration at this time is shown in figure 5(c). It is found from figure 5(c) that, even when the organic acid prepared as described above is added to FAD-GDH, the FAD-GDH has the substrate specificity to glucose which is approximately equal to that of PQQ-GDH.

As described above, a biosensor which has a reduced blanking current value and a high substrate specificity to glucose and thereby can perform highly precise measurement can be fabricated by adding the organic acid into the reagent layer.

### (Example 2)

Next, the sensor response characteristics in the case where an amino acid is added to the reagent layer 105 will be described.

A biosensor of this second example is fabricated according to the same procedure as that of the first example. Then, taurine as an example of amino acid is further added to the reagent layer 105 of the biosensor.

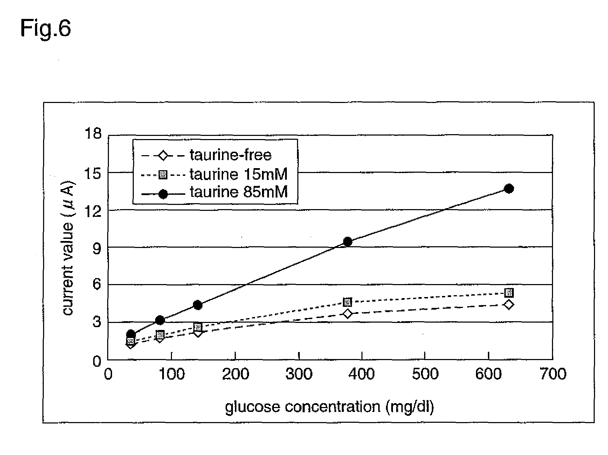

Figure 6 shows the response characteristics of current in the case where taurine is added to the reagent layer 105 by 0mM, 15mum, and 85mM, respectively.

As can be seen from figure 6, the sensor response characteristics are dramatically increased as the additive amount of taurine is increased.

Accordingly, a biosensor which is excellent in the substrate specificity to glucose and thereby performs highly precise measurement can be realized by adding taurine to the reagent layer 105.

### (Example 3)

Next, the sensor response characteristics in the case where a sugar alcohol is added to the reagent layer 105 will be described.

A biosensor of this third example is fabricated according to the same procedure as that of the first example. Then, maltitol or lactitol as an example of sugar alcohol is further added to the reagent layer 105 of the biosensor. Whole blood having a glucose concentration of 80mg/dL is used as a sample solution.

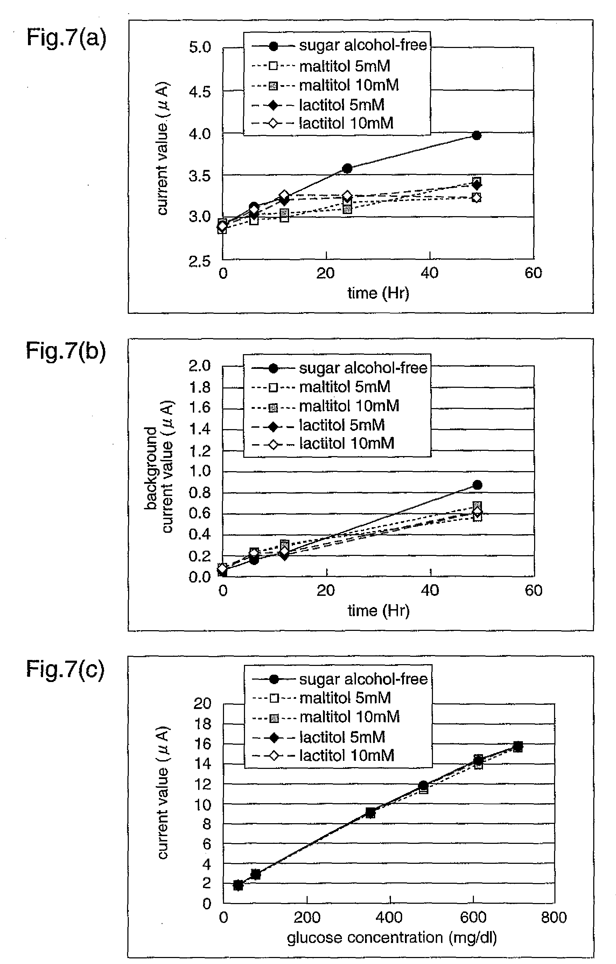

Figure 7(a) shows the preservation characteristics under a high temperature and humidity environment (temperature of 40°C, humidity of 80%) in the case where maltitol or lactitol is added to the reagent layer 5 by 0mM, 5mM, and 10mM, respectively.

As can be seen from figure 7(a), an increase in the current value due to the high temperature and humidity environment can be suppressed when maltitol or lactitol is added, and thereby the preservation characteristics of the sensor are evidently improved.

Figure 7(b) shows the response characteristics of background current under the hot and humid environment (temperature of 40°C, humidity of 80%) in the case where maltitol or lactitol is added to the reagent layer 5 by 0mM, 5mM, and 10mM, respectively.

As can be seen from figure 7(b), an increase in the background current value under the high temperature and humidity environment can be suppressed by adding maltitol or lactitol, and thereby the preservation characteristics of the sensor are improved.

Figure 7(c) shows the response characteristics of current in the case where maltitol or lactitol is added to the reagent layer 5 by 0mM 5mM, and 10mM, respectively.

As can be seen from figure '7(c), even when maltitol or lactitol is added, a favorable linearity can be maintained without deteriorating the response characteristics of the sensor to the glucose concentration.

Consequently, a biosensor which has a favorable linearity and an excellent preservation characteristics and thereby performs highly precise measurement can be realized by adding maltitol or lactitol as a sugar alcohol.

### (Example 4)

Next, the influence by hematocrit will be described.

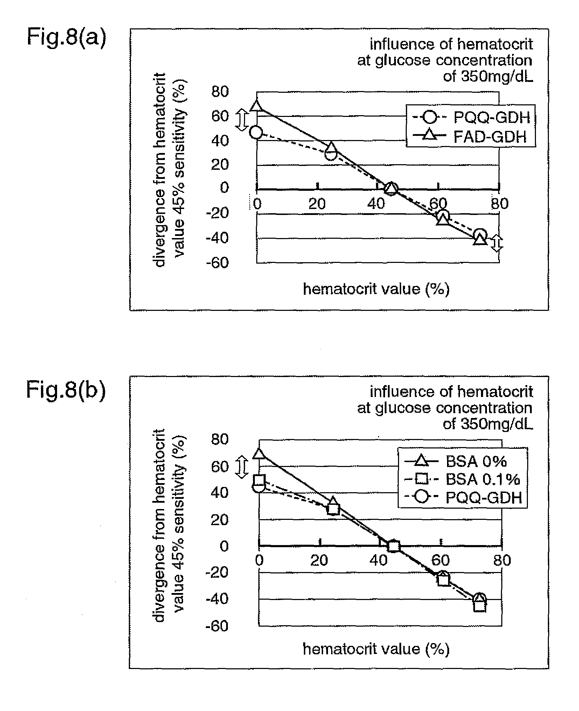

Figure 8(a) is a graph illustrating the influence by hematocrit to the sensor response characteristics in the case where whole blood having a glucose concentration of 350mg/dL is used as a sample solution. In figure 8(a), the ordinate shows the deviation from the sensitivity with the hematocrit value of 45%, and the abscissa shows the hematocrit value.

When the sensor response characteristics in the case of using PQQ-GDH and the sensor response characteristics in the case of using FAD-GDH are compared with each other, no difference in the sensor response characteristics due to the influence by hematocrit is shown in a range where the hematocrit value is about 25 to 75%. A remarkable difference due to the influence by hematocrit is shown in a low concentration range where the hematocrit value is 25% or below, and thus it is recognized that the influence by hematocrit is particularly considerable when FAD-GDH is used.

Thus, the sensor response characteristics in the case where FAD-GDH is used as an enzyme are more likely subjected to the influence by hematocrit in the low hematocrit concentration range as compared with the case where PQQ-GDH is used. Therefore, a biosensor having an excellent preservation stability, which is not likely to be affected by hematocrit, is desired.

So, BSA as a solubilized protein is added to the reagent layer 105. Figure 8(b) is a diagram illustrating the influence by hematocrit to the sensor response characteristics in the case where BSA is added to the reagent layer.

When BSA of 0.1wt% is added to the reagent layer 105, the influence by the hematocrit can be reduced to approximately the same degree as that for PQQ-GDH in the low hematocrit concentration range.

In this way, the influence by hematocrit is suppressed to the same degree as that of the conventional biosensor using PQQ-GDH, and thereby a biosensor which is excellent in preservation stability and substrate specificity to glucose can be fabricated.

### APPLICABILITY IN INDUSTRY

A biosensor of the present invention is applicable as an enzyme sensor for glucose, which is excellent in the substrate specificity and is able to perform highly accurate measurement.

## Claims

1. A biosensor which, having a reagent layer including a reagent which reacts specifically with a specific component in a sample solution, measures the concentration of the specific component in the sample solution,
said reagent layer including glucose dehydrogenase having flavin adenine dinucleotide as a coenzyme, and an organic acid or its salt having at least one carboxyl group in a molecule thereof.

2. A biosensor as defined in Claim 1, wherein the concentration of the specific component in the sample solution is measured using electrodes including at least a working electrode and a counter electrode, which electrodes are formed on an insulating substrate.

3. A biosensor as defined in Claim 2, wherein the reagent layer, including an electron carrier, is formed on the electrodes.

4. A biosensor as defined in Claim 2, wherein the reagent layer, including an electron carrier, is formed so that the electrodes are disposed in a diffusion area wherein the reagent of the reagent layer is dissolved in the sample solution and diffused.

5. A biosensor as defined in Claim 1, wherein the organic acid is aliphatic carboxylic acid, carbocyclic carboxylic acid, or heterocyclic carboxylic acid, or a substitution or derivative thereof.

6. A biosensor as defined in Claim 5, wherein the organic acid or its salt is any of citric acid, citrate, phthalic acid, and phthalate, or a combination of these.

7. A biosensor which, having a reagent layer including a reagent which reacts specifically with a specific component in a sample solution, measures the concentration of the specific component in the sample solution,
said reagent layer including glucose dehydrogenase having flavin adenine dinucleotide as a coenzyme, and an organic acid or its salt having at least one amino group or carbonyl group in a molecule thereof.

8. A biosensor as defined in Claim 7, wherein the concentration of the specific component in the sample solution is measured using electrodes including at least a working electrode and a counter electrode, which electrodes are formed on an insulating substrate.

9. A biosensor as defined in Claim 8, wherein the reagent layer, including an electron carrier, is formed on the electrodes.

10. A biosensor as defined in Claim 8, wherein the reagent layer, including an electron carrier, is formed so that the electrodes are disposed in a diffusion area wherein the reagent of the reagent layer is dissolved in the sample solution and diffused.

11. A biosensor as defined in Claim 7, wherein the organic acid is amino acid, or a substitution or derivative thereof.

12. A biosensor as defined in Claim 11, wherein the organic acid is taurine.

13. A biosensor which, having a reagent layer including a reagent which reacts specifically with a specific component in a sample solution, measures the concentration of the specific component in the sample solution,
said reagent layer including glucose dehydrogenase having flavin adenine dinucleotide as a coenzyme, and a sugar alcohol.

14. A biosensor as defined in Claim 13, wherein the concentration of the specific component in the sample solution is measured using electrodes including at least a working electrode and a counter electrode, which electrodes are formed on an insulating substrate.

15. A biosensor as defined in Claim 14, wherein the reagent layer, including an electron carrier, is formed on the electrodes.

16. A biosensor as defined in Claim 14, wherein the reagent layer, including an electron carrier, is formed so that the electrodes are disposed in a diffusion area wherein the reagent of the reagent layer is dissolved in the sample solution and diffused.

17. A biosensor as defined in Claim 13, wherein the sugar alcohol is chain polyhydric alcohol or cyclic sugar alcohol, or a substitution or derivative thereof.

18. A biosensor as defined in Claim 17, wherein the sugar alcohol is either or both of maltitol and lactitol.

19. A biosensor which, having a reagent layer including a reagent which reacts specifically with a specific component in a sample solution, measures the concentration of the specific component in the sample solution,
said reagent layer including glucose dehydrogenase having flavin adenine dinucleotide as a coenzyme, and a solubilized protein.

20. A biosensor as defined in Claim 19, wherein the concentration of the specific component in the sample solution is measured using electrodes including at least a working electrode and a counter electrode, which electrodes are formed on an insulating substrate.

21. A biosensor as defined in Claim 20, wherein the reagent layer, including an electron carrier, is formed on the electrodes.

22. A biosensor as defined in Claim 20, wherein the reagent layer, including an electron carrier, is formed so that the electrodes are disposed in a diffusion area wherein the reagent of the reagent layer is dissolved in the sample solution and diffused.

23. A biosensor as defined in Claim 19, wherein the solubilized protein is bovine serum albumin (BSA), egg albumin, gelatin, or collagen.

24. A biosensor which, having a reagent layer including a reagent which reacts specifically with a specific component in a sample solution, measures the concentration of the specific component in the sample solution,
said reagent layer including glucose dehydrogenase having flavin adenine dinucleotide as a coenzyme, and at least two additive agents among an organic acid or its salt having at least one carboxyl group in a molecule thereof, an organic acid or its salt having at least one amino group or carbonyl group in a molecule thereof, a sugar alcohol, and a solubilized protein.

25. A biosensor as defined in Claim 24, wherein the concentration of the specific component in the sample solution is measured using electrodes including at least a working electrode and a counter electrode, which electrodes are formed on an insulating substrate.

26. A biosensor as defined in Claim 25, wherein the reagent layer, including an electron carrier, is formed on the electrodes.

27. A biosensor as defined in Claim 25, wherein the reagent layer, including an electron carrier, is formed so that the electrodes are disposed in a diffusion area wherein the reagent of the reagent layer is dissolved in the sample solution and diffused.
